(19) Europäisches Patentamt
European Patent Office
Office européen des brevets

(11) EP 1 350 780 A1

(12) **EUROPÄISCHE PATENTANMELDUNG**

(43) Veröffentlichungstag:
**08.10.2003 Patentblatt 2003/41**

(51) Int Cl.7: **C07C 25/22**, C07C 49/697, C09K 19/32

(21) Anmeldenummer: **03004926.6**

(22) Anmeldetag: **07.03.2003**

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**AL LT LV MK RO**

(30) Priorität: **04.04.2002 DE 10214938**

(71) Anmelder: **MERCK PATENT GmbH**
**64293 Darmstadt (DE)**

(72) Erfinder:
- **Leitzau, Lars, Dr.**
  **64295 Darmstadt (DE)**
- **Klasen-Memmer, Melanie, Dr.**
  **67259 Heuchelheim (DE)**
- **Bremer, Matthias, Dr.**
  **64295 Darmstadt (DE)**

(54) **Fluorierte Indene und 1,7-Dihydroindacene mit negativer dielektrischer Anisotropie**

(57) Die Erfindung betrifft fluorierte Indene und 1,7-Dihydroindacene der allgemeinen Formel (I) bzw. (II) mit negativer dielektrischer Anisotropie ($\Delta\varepsilon < 0$)

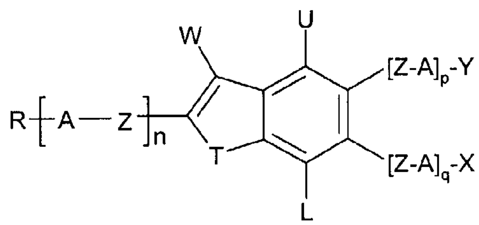

(I)

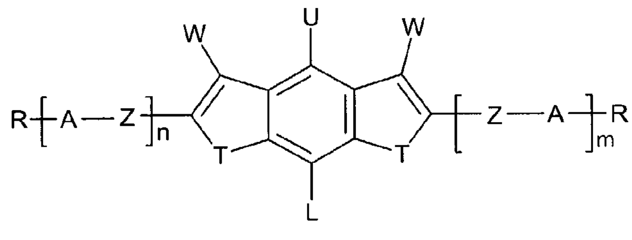

(II)

worin
T, A, R, L, X, Y, U, W, Z, n, m, p und q die in den Ansprüchen definierte Bedeutung haben.

EP 1 350 780 A1

Printed by Jouve, 75001 PARIS (FR)

## Beschreibung

Die Erfindung betrifft fluorierte Indene und 1,7-Dihydroindacene mit negativem $\Delta\varepsilon$ und deren Verwendung in flüssigkristallinen Medien.

Flüssigkristalle haben ein breites Anwendungsfeld gefunden, seitdem vor etwa 30 Jahren die ersten kommerziell anwendbaren flüssigkristallinen Verbindungen aufgefunden wurden. Bekannte Anwendungsgebiete sind insbesondere Anzeigedisplays für Uhren oder Taschenrechner, sowie große Anzeigetafeln, wie sie in Bahnhöfen, Flughäfen und Sportarenen verwendet werden. Weitere Anwendungsgebiete sind Displays von tragbaren Computern oder Navigationssystemen sowie Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante $\varepsilon$ des flüssigen Kristalls ist also für die beiden Orientierungen verschieden groß. Substanzen, deren Dielektrizitätskonstante bei senkrechter Orientierung der Molekül-Längsachsen zu den Kondensatorplatten größer ist als bei paralleler Anordnung, werden als dielektrisch positiv bezeichnet. Die meisten flüssigen Kristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab. Bei kleinen Differenzen sind höhere Schaltspannungen erforderlich als bei großen. Durch Einführen geeigneter polarer Gruppen wie Nitrilgruppen (-CN) oder Fluor in die Flüssigkristallmoleküle lässt sich ein weiter Bereich von Arbeitsspannungen realisieren.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment. Eine Verbesserung der Blickwinkelabhängigkeit ist nur mit sehr hohem Aufwand, z.B. Filmkompensation, möglich. Bei den am weitesten verbreiteten TN-Zellen (nach englisch "twisted nematic", verdrillt nematisch) ist eine nur etwa 3 bis 10 µm dicke flüssigkristalline Schicht zwischen zwei ebenen Glasplatten angeordnet, auf die jeweils eine elektrisch leitende, transparente Schicht aus Zinnoxid oder Indium-Zinnoxid als Elektrode aufgedampft ist. Zwischen diesen Filmen und der flüssigkristallinen Schicht befindet sich eine ebenfalls transparente Orientierungsschicht, die meist aus einem Kunststoff (z.B. Polyimiden) besteht. Sie dient dazu, durch Oberflächenkräfte die Längsachsen der benachbarten kristallinen Moleküle in eine Vorzugsrichtung zu bringen, so dass sie im spannungsfreien Fall einheitlich mit der gleichen Orientierung flach oder mit demselben kleinen Anstellwinkel auf der Innenseite der Displayfläche aufliegen. Auf der Außenseite des Displays sind zwei Polarisationsfolien, die nur linear polarisiertes Licht ein- und austreten lassen, in bestimmten Anordnungen aufgeklebt.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase zu erreichen sowie kurze Schaltzeiten und niedrige Schwellenspannungen. Schwierigkeiten bereitet jedoch noch die starke Blickwinkelabhängigkeit bei Flüssigkristallanzeigen, wie sie beispielsweise für Laptops verwendet werden. Die beste Abbildungsqualität lässt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Im feldfreien Zustand werden die Moleküle senkrecht zur Glasfläche des Displays orientiert. Derartige Displays werden als VA-TFT-Displays bezeichnet (von englisch "vertical aligned"). Bei angelegtem Feld orientieren sich die Moleküle mit ihren Längsachsen senkrecht zu den Feldlinien. Durch die sogenannte Multidomain-Technik konnte eine Verbesserung der Blickwinkelabhängigkeit erreicht werden.

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

Aufgabe der Erfindung ist daher, flüssigkristalline Phasen bildende Verbindungen mit vorteilhaften Eigenschaften zur Verfügung zu stellen.

Diese Aufgabe wird gelöst durch Indene und 1,7-Dihydroindacene der allgemeinen Formel (I) bzw. (II)

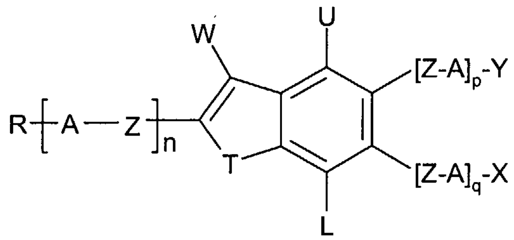

(I)

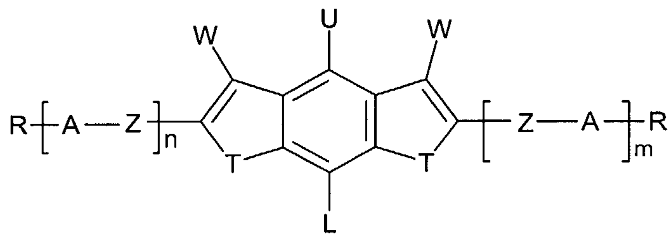

(II)

worin bedeuten:

T jeweils unabhängig voneinander $>CF_2$, $>CHF$, $>CH_2$, $>C=O$,

A jeweils unabhängig voneinander 1,4-Phenylen, worin =CH- ein- oder zweimal durch =N- ersetzt sein kann, und das ein- bis viermal unabhängig voneinander mit Halogen (-F, -Cl, -Br, -I), -CN, $-CH_3$, $-CH_2F$, $-CHF_2$, $-CF_3$, $-OCH_3$, $-OCH_2F$, $-OCHF_2$ oder $-OCF_3$ substituiert sein kann, 1,4-Cyclohexylen, 1,4-Cyclohexenylen oder 1,4-Cyclohexadienylen, worin $-CH_2-$ ein- oder zweimal unabhängig voneinander durch -O- oder -S- ersetzt sein kann, und die ein oder mehrfach durch Halogen substituiert sein können,

R jeweils unabhängig voneinander Wasserstoff, einen unsubstituierten, einen einfach durch $-CF_3$ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere $CH_2$-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN, -NCS, $-SF_5$, $-CF_3$, $-OCF_3$, $-OCHF_2$ oder $-OCH_2F$,

L Wasserstoff oder Halogen,

X Wasserstoff, einen unsubstituierten, einen einfach durch $-CF_3$ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere $CH_2$-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN oder -NCS,

Y, U, W unabhängig voneinander Wasserstoff, einen unsubstituierten, einen einfach durch $-CF_3$ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere $CH_2$-Gruppen jeweils unabhängig voneinander durch -O-, -S-, -CO-, -COO-, -OCO-oder -OCO-O- so ersetzt sein können, dass Heteroatome nicht direkt benachbart sind,

Z jeweils unabhängig voneinander eine Einfachbindung, $-CF_2O-$, $-OCF_2-$, $-CH_2CH_2-$, $-CF_2CF_2-$, -C(O)O-, -OC(O)-, $-CH_2O-$, $-OCH_2-$, -CF=CH-, -CH=CF-, -CF=CF-, -CH=CH- oder $-C\equiv C-$,

n, m unabhängig voneinander 0, 1, 2 oder 3,

p, q unabhängig voneinander 0, 1, 2 oder 3,

mit der Maßgabe, dass in Formel (I) L = -F ist, wenn
T = $>CH_2$ ist und p und q jeweils 0 bedeuten.

Die Verbindungen besitzen sämtlich ein negatives $\Delta\varepsilon$ und eignen sich daher für eine Verwendung in VA-TFT-Displays. Sie zeigen eine sehr gute Verträglichkeit mit den üblichen, in Flüssigkristallmischungen für Displays verwendeten Substanzen.

Durch die Fluorsubstituenten im Inden- bzw. 1,7-Dihydroindacengerüst wird ein Dipolmoment senkrecht zur Moleküllängsachse erzeugt, das gegebenenfalls durch geeignete Substituenten in den Flügeleinheiten ZAZAR weiter verstärkt werden kann. Im feldfreien Zustand richten sich die Verbindungen der Formel (I) bzw. (II) mit ihrer Moleküllängsachse senkrecht zur behandelten oder beschichteten Glasfläche eines Displays aus.

In den allgemeinen Formeln (I) und (II) sind A bevorzugt unabhängig voneinander gegebenenfalls substituiertes 1,4-Phenylen, gegebenenfalls substituiertes 1,4-Cyclohexylen, worin $-CH_2-$ ein- oder zweimal durch -O- ersetzt sein kann, oder gegebenenfalls substituiertes 1,4-Cyclohexenylen.

Besonders bevorzugt sind A unabhängig voneinander

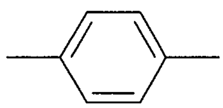 , 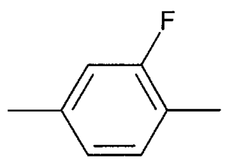 , 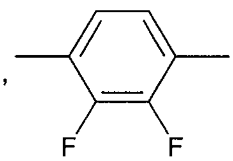 ,

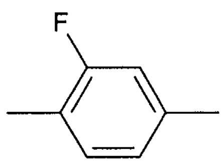 , 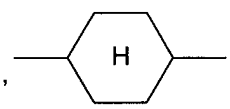 , 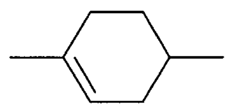 ,

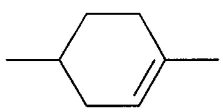 , 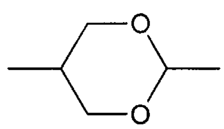 oder 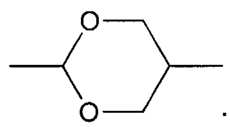 .

Wenn L Halogen ist, ist es bevorzugt Fluor oder Chlor, besonders bevorzugt Fluor.

R, X, Y, U und W in den allgemeinen Formeln (I) und (II) können jeweils unabhängig voneinander ein Alkylrest und/oder einen Alkoxyrest mit 1 bis 15 C-Atomen sein, der geradkettig oder verzweigt sein kann. Vorzugsweise ist er geradkettig, hat 1, 2, 3, 4, 5, 6 oder 7 C-Atome und ist demnach bevorzugt Methyl, Ethyl, Propyl, Butyl, Pentyl, Hexyl, Heptyl, Methoxy, Ethoxy, Propoxy, Butoxy, Pentoxy, Hexoxy oder Heptoxy.

R, X, Y, U und W können jeweils unabhängig voneinander Oxaalkyl sein, vorzugweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4-, 5- oder 6-Oxaheptyl.

R, X, Y, U und W können jeweils unabhängig voneinander ein Alkenylrest mit 2 bis 15 C-Atomen sein, der geradkettig oder verzweigt sein kann. Vorzugsweise ist er geradkettig und hat 2 bis 7 C-Atome. Er ist demnach insbesondere Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl.

R, X, Y, U und W können jeweils unabhängig voneinander ein Alkylrest mit 1 bis 15 C-Atomen sein, in dem eine $CH_2$-Gruppe durch -O- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser geradkettig und hat 2 bis 6 C-Atome.

R, X, Y, U und W können jeweils unabhängig voneinander ein Alkylrest mit 1 bis 15 C-Atomen sein, in dem eine $CH_2$-Gruppe durch unsubstituiertes oder substituiertes -CH=CH- und eine benachbarte $CH_2$-Gruppe durch -CO-,

-CO-O- oder -O-CO- ersetzt ist, wobei dieser geradkettig oder verzweigt sein kann. Vorzugsweise ist er geradkettig und hat 4 bis 13 C-Atome.

R, X, Y, U und W können jeweils unabhängig voneinander ein einfach durch -CN oder $-CF_3$ substituierter Alkylrest mit 1 bis 15 C-Atomen oder Alkenylrest mit 2 bis 15 C-Atomen sein, wobei diese vorzugsweise geradkettig sind. Die Substitution durch -CN oder $-CF_3$ ist in beliebiger Position.

R, X, Y, U und W können jeweils unabhängig voneinander ein mindestens einfach durch Halogen substituierter Alkylrest mit 1 bis 15 C-Atomen oder Alkenylrest mit 2 bis 15 C-Atomen sein, wobei diese Reste vorzugsweise geradkettig sind und Halogen vorzugsweise -F oder -Cl ist. Bei Mehrfachsubstitution ist Halogen vorzugsweise -F. Die resultierenden Reste schließen auch perfluorierte Rest wie $-CF_3$ ein. Bei Einfachsubstitution kann der Fluor- oder Chlorsubstituent in beliebiger Position sein, vorzugsweise ist er in ω-Position.

R, X, Y, U und W können jeweils unabhängig voneinander ein Alkylrest sein, in dem zwei oder mehr $CH_2$-Gruppen durch -O- und/oder -CO-O- ersetzt sind, wobei dieser geradkettig oder verzweigt sein kann. Vorzugsweise ist er verzweigt und hat 3 bis 12 C-Atome.

Bevorzugt sind R in den allgemeinen Formeln (I) und (II) jeweils unabhängig voneinander ein Alkylrest, Alkoxyrest oder Alkenylrest mit 1 bis 7 bzw. 2 bis 7 C-Atomen.

Bevorzugte Brückengruppen Z in den Verbindungen der allgemeinen Formeln (I) und (II) sind jeweils unabhängig voneinander eine Einfachbindung, $-CF_2O-$, $-OCF_2-$, $-CF_2CF_2-$, -CH=CH-, -CF=CH-, -CH=CF- oder -CF=CF-.

Bevorzugt sind U und W in Formel (I) bzw. Formel (II) Wasserstoff.

Bevorzugte Substituenten X, Y in der allgemeinen Formel (I) sind Wasserstoff, ein Alkylrest, Alkoxyrest oder Alkenylrest mit 1 bis 7 bzw. 2 bis 7 C-Atomen, wie die oben genannten, geradkettigen Alkyl-, Alkoxy- und Alkenylreste.

X kann zusätzlich Halogen, -CN, -SCN oder -NCS sein. Bevorzugt ist es Halogen, insbesondere Fluor.

Die Verbindungen der allgemeinen Formel (I) bzw. (II) weisen am Inden- bzw. 1,7-Dihydroindacengerüst mindestens einen lateralen Fluor-Substituenten auf, das heißt T, L und/oder X in Formel (I) bzw. T und/oder L in Formel (II) enthalten mindestens ein Fluor. Ist in Formel (I) bzw. Formel (II)

$$T \neq \rangle C=O ,$$

so enthalten T, L und X in Formel (I) bevorzugt 2 bis 4 Fluoratome bzw. enthalten T und L in Formel (II) bevorzugt 3 bis 5 Fluoratome.

Bevorzugte Indene der allgemeinen Formel (I) weisen ein oder zwei Cyclen A auf, das heißt n + p + q = 1 oder 2.

Bevorzugte 1,7-Dihydroindacene der allgemeinen Formel (II) weisen bevorzugt ein oder zwei Cyclen A auf, das heißt n + m = 1 oder 2.

Beispiele für erfindungsgemäße Indene und 1,7-Dihydroindacene sind die nachstehenden Verbindungen:

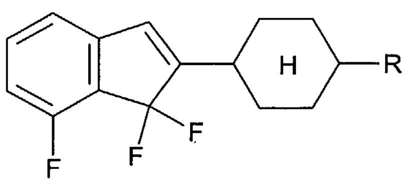

(Ia)

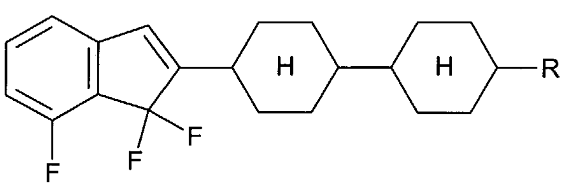

(Ib)

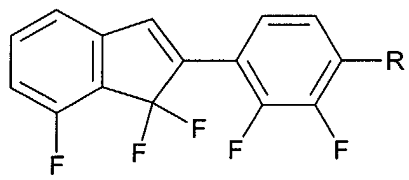

(Ic)

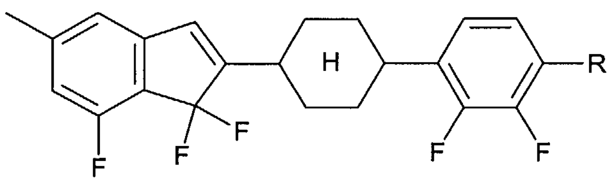

(Id)

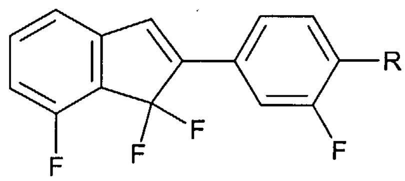

(Ie)

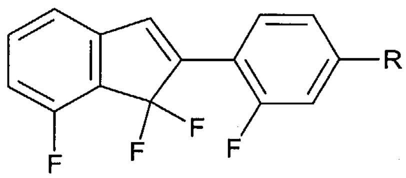

(If)

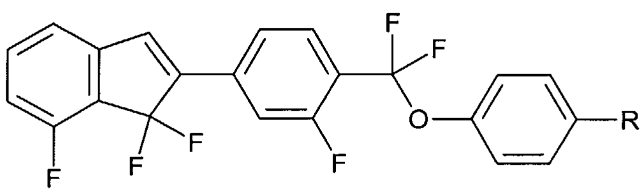

(Ig)

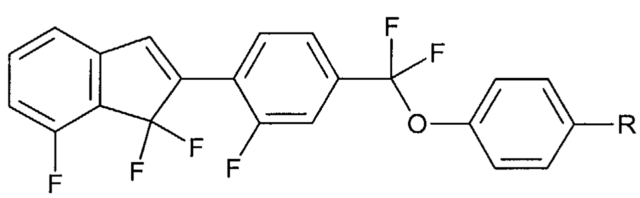

(Ih)

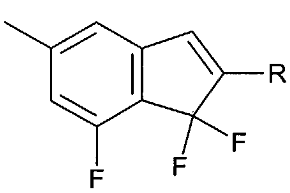

(Ii)

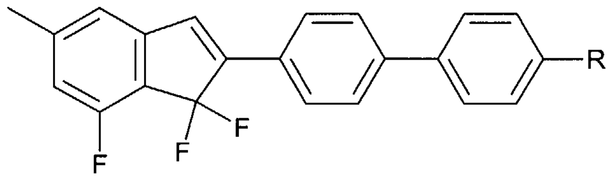

(Ij)

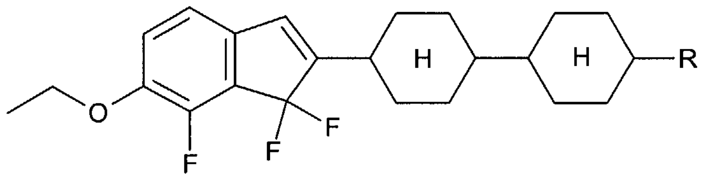

(Ik)

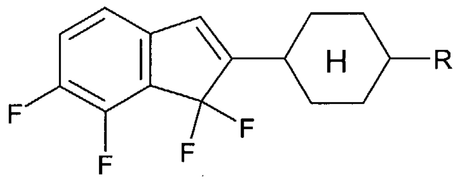

(Il)

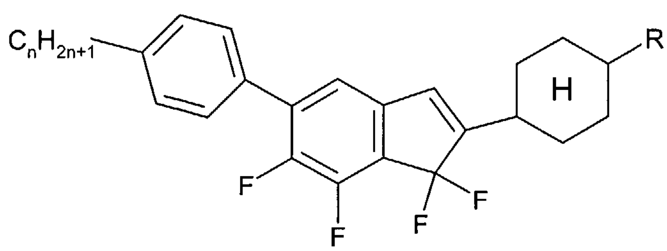

(Im)

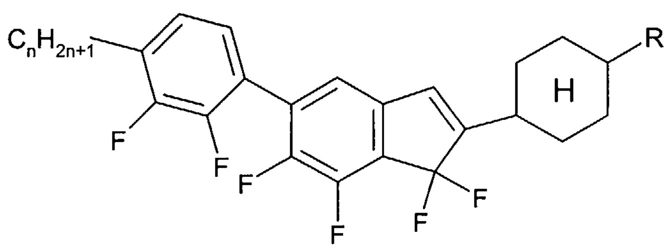

(In)

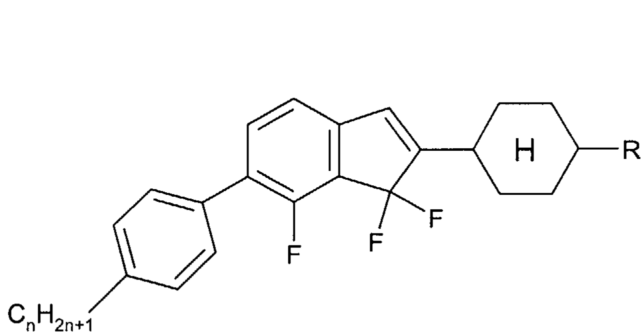

(Io)

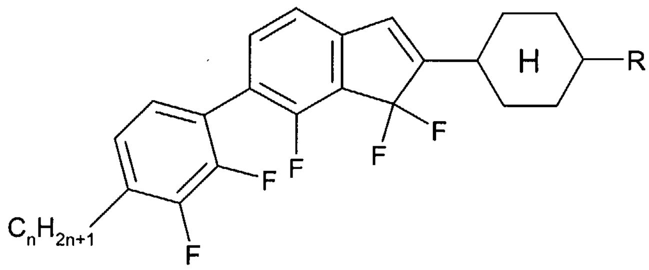

(Ip)

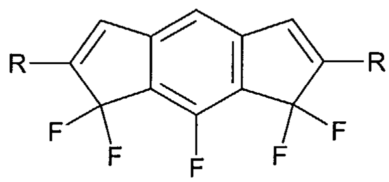

(IIa)

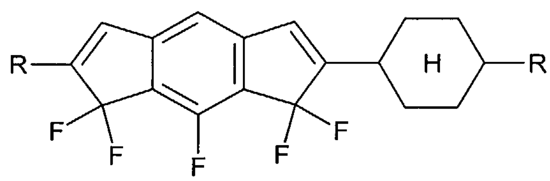

(IIb)

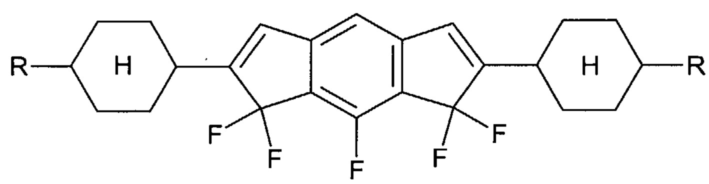

(IIc)

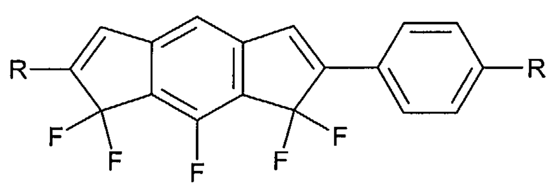

(IId)

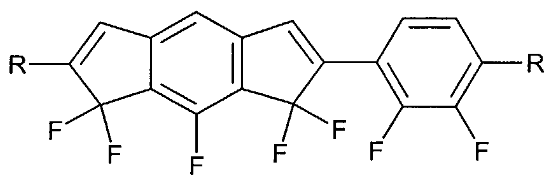

(IIe)

Darin sind R jeweils unabhängig voneinander ein Alkyl-, Alkenyl- oder Alkoxyrest mit 1 bis 7 bzw. 2 bis 7 C-Atomen und n bedeutet 1, 2, 3, 4, 5, 6 oder 7.

Die Verbindungen der allgemeinen Formeln (I) und (II) werden nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der allgemeinen Formeln (I) und (II) umsetzt.

Eine beispielhafte Synthese ist im folgenden dargestellt. Die Synthese kann durch die Wahl geeigneter Ausgangsprodukte an die jeweils gewünschten Verbindungen der allgemeinen Formeln (I) und (II) angepasst werden.

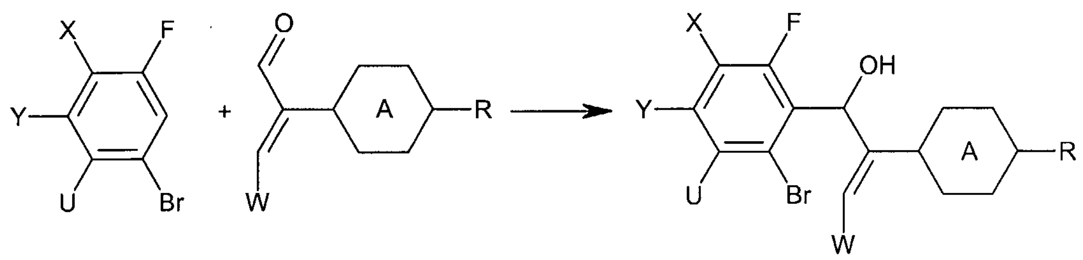

**a** **b** **c**

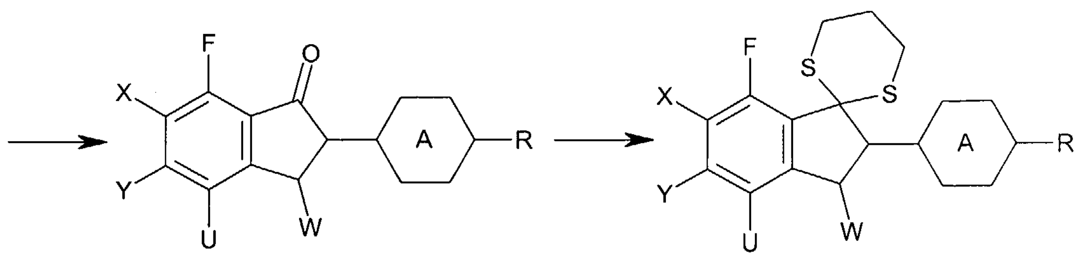

**d** **e**

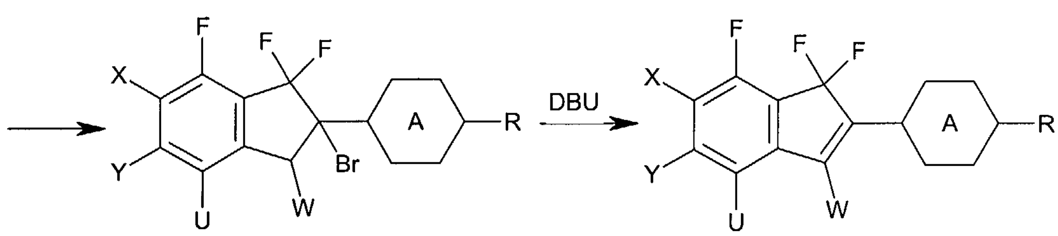


**f** **g**

Ausgehend vom 3-Bromfluorbenzol **a** wird durch Umsetzung mit dem α, β-ungesättigten Aldehyd **b** in Gegenwart von Lithiumdiisopropylamid (LDA) die Verbindung c erhalten. Diese reagiert unter Palladiumkatalyse in Gegenwart von Triethylamin unter Ringschluss zum Indanon **d.** Aus dem Indanon **d** und 1,3-Propandithiol wird in Gegenwart von $BF_3$-Diethylether das entsprechende Dithian **e** erhalten. Dieses wird mit 1,3-Dibrom-5,5-dimethylhydantoin (DBH) und HF in Pyridin zum Trifluorbromindan **f** umgesetzt. Eliminierung von HBr in Gegenwart von Diazabicycloundecen (DBU) ergibt das Trifluorinden **g**.

Analog wird das 1,7-Dihydroindacen (II) erhalten, wenn an Stelle des (substituierten) 3-Bromfluorbenzol von 3,5-Dibromfluorbenzol ausgegangen wird.

Die dargestellten Reaktionen sind nur als beispielhaft aufzufassen. Der Fachmann kann entsprechende Variationen der vorgestellten Synthesen vornehmen sowie auch andere geeignete Synthesewege beschreiten, um Verbindungen der Formeln (I) oder (II) zu erhalten.

Wie bereits erwähnt, können die Verbindungen der allgemeinen Formeln (I) und (II) zur Herstellung flüssigkristalliner Mischungen verwendet werden. Gegenstand der Erfindung ist daher auch ein flüssigkristallines Medium mit mindestens zwei flüssigkristallinen Verbindungen, umfassend mindestens eine Verbindung der allgemeinen Formeln (I) und/oder (II).

Gegenstand der vorliegenden Erfindung sind auch flüssigkristalline Medien enthaltend neben einer oder mehreren erfindungsgemäßen Verbindungen der Formeln (I) und/oder (II) als weitere Bestandteile 2 bis 40, vorzugsweise 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäurephenyl- oder -cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln (III), (IV), (V), (VI) und (VII) charakterisieren:

$$R'\text{-}L\text{-}E\text{-}R'' \qquad \text{(III)}$$

$$R'\text{-}L\text{-}COO\text{-}E\text{-}R'' \qquad \text{(IV)}$$

$$R'\text{-}L\text{-}OOC\text{-}E\text{-}R'' \qquad \text{(V)}$$

$$R'\text{-}L\text{-}CH_2CH_2\text{-}E\text{-}R'' \qquad \text{(VI)}$$

$$R'\text{-}L\text{-}CF_2O\text{-}E\text{-}R'' \qquad \text{(VII)}$$

In den Formeln (III), (IV), (V), (VI) und (VII) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (III), (IV), (V), (VI) und (VII), worin L und E ausgewählt sind aus der Gruppe Cyc und Phe und gleichzeitig eine oder mehrere Komponenten ausgewählt aus den Verbindungen der Formeln (III), (IV), (V), (VI) und (VII), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (III), (IV), (V), (VI) und (VII), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (III), (IV), (V), (VI) und (VII) jeweils unabhängig voneinander Alkyl, Alkenyl, Alkoxy, Alkoxyalkyl, Alkenyloxy oder Alkanoyloxy mit bis zu 8 Kohlenstoffatomen. Im Folgenden wird diese kleinere Untergruppe Gruppe A genannt und die Verbindungen werden mit den Teilformeln (IIIa), (IVa), (Va), (VIa) und (VIIa) bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln (III), (IV), (V), (VI) und (VII) bedeutet E

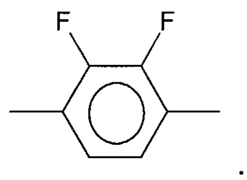

In den Verbindungen der Gruppe B, die mit den Teilformeln (IIIb), (IVb), (Vb), (VIb) und (VIIb) bezeichnet werden, haben R' und R" die bei den Verbindungen der Teilformeln (IIIa) bis (VIIa) angegebene Bedeutung und sind vorzugsweise Alkyl, Alkenyl, Alkoxy oder Alkoxyalkyl.

In einer weiteren kleineren Untergruppe der Verbindungen der Formeln (III), (IV), (V), (VI) und (VII) bedeutet R" -CN. Diese Untergruppe wird im Folgenden als Gruppe C bezeichnet und die Verbindungen dieser Untergruppe werden entsprechend mit Teilformeln (IIIc), (IVc), (Vc), (VIc) und (VIIc) beschrieben. In den Verbindungen der Teilformeln (IIIc), (IVc), (Vc), (VIc) und (VIIc) hat R' die bei den Verbindungen der Teilformeln (IIIa) bis (VIIa) angegebene Bedeutung und ist vorzugsweise Alkyl, Alkoxy oder Alkenyl.

Neben den bevorzugten Verbindungen der Gruppen A, B und C sind auch andere Verbindungen der Formeln (III), (IV), (V), (VI) und (VII) mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der allgemeinen Formeln (I) und/oder (II) vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise:

Gruppe A: 0 bis 90%, vorzugsweise 20 bis 90%, insbesondere 30 bis 90%
Gruppe B: 0 bis 80%, vorzugsweise 10 bis 80%, insbesondere 10 bis 70%
Gruppe C: 0 bis 80%, vorzugsweise 5 bis 80%, insbesondere 5 bis 50%

wobei die Summe der Massenanteile der in den jeweiligen erfindungsgemäßen Medien enthaltenen Verbindungen aus den Gruppen A und/oder B und/oder C vorzugsweise 5 bis 90% und insbesondere 10 bis 90% beträgt.

Die erfindungsgemäßen Medien enthalten vorzugsweise 1 bis 40%, insbesondere 5 bis 30% an erfindungsgemäßen Verbindungen der Formeln (I) und/oder (II). Weiterhin bevorzugt sind Medien, enthaltend mehr als 40%, insbesondere 45 bis 90% an erfindungsgemäßen Verbindungen der Formeln (I) und/oder (II). Die Medien enthalten vorzugsweise drei, vier oder fünf erfindungsgemäße Verbindungen der Formeln (I) und/oder (II).

Beispiele für die Verbindungen der Formeln (III), (IV), (V), (VI) und (VII) sind die nachstehend aufgeführten Verbindungen:

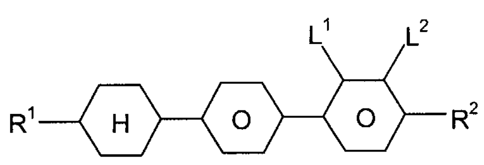

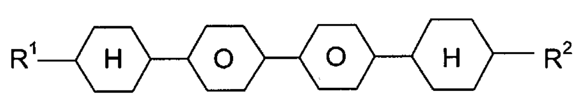

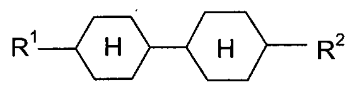

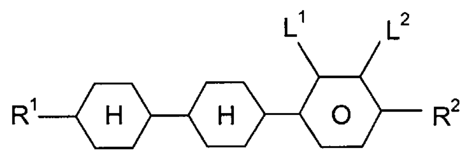

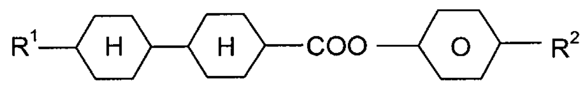

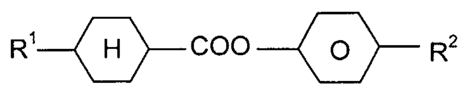

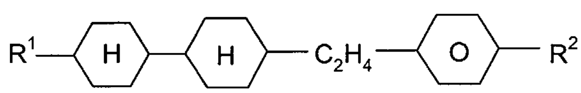

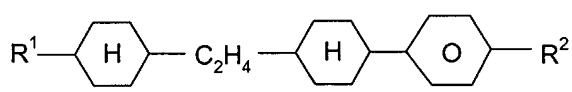

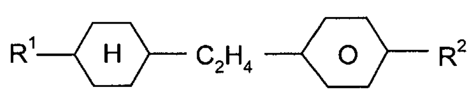

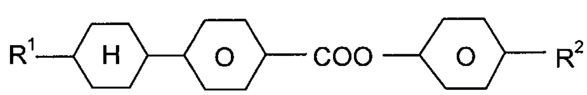

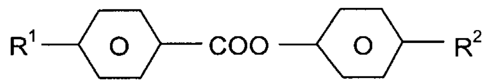

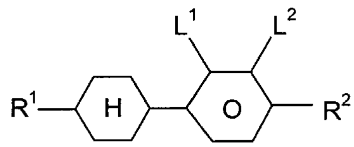

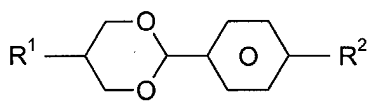

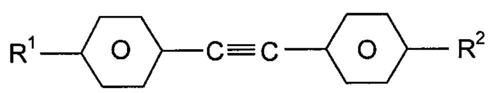

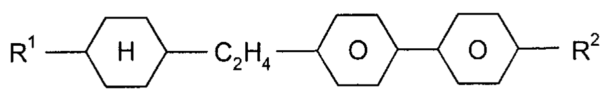

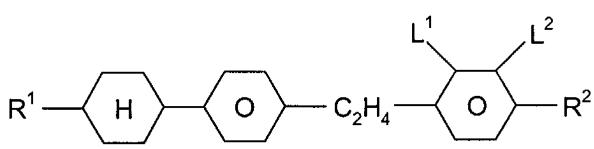

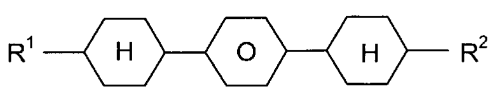

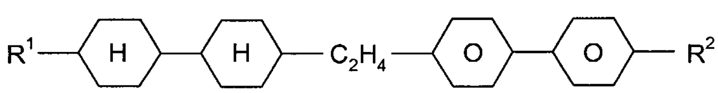

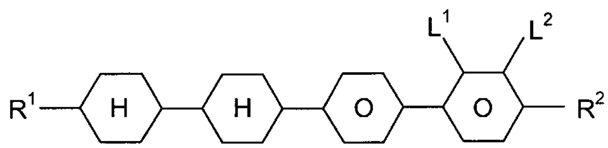

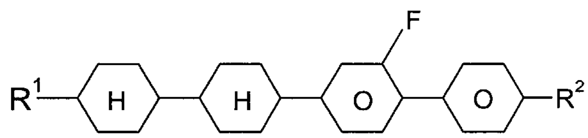

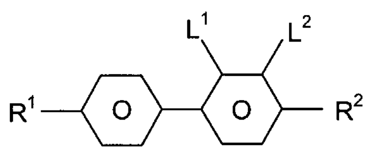

mit $R^1$, $R^2$ = $-C_nH_{2n+1}$, $-C_nH_{2n-1}$, oder $-OC_nH_{2n+1}$ und n = 1 bis 8, und $L^1$, $L^2$ =-H oder -F,

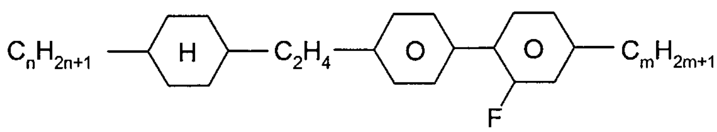

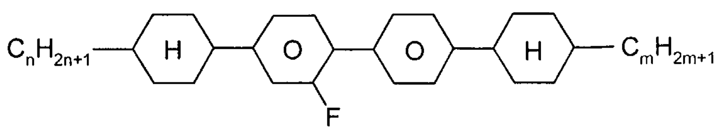

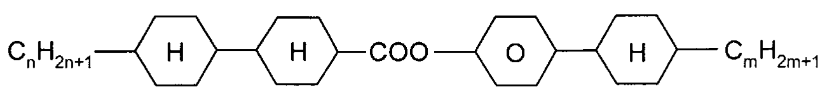

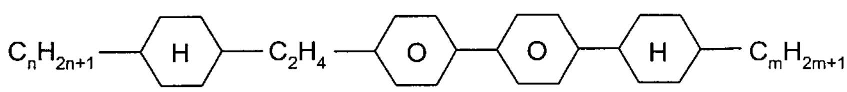

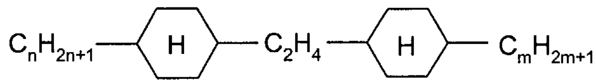

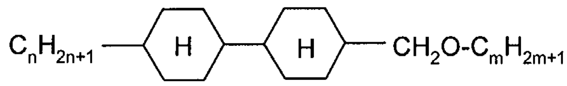

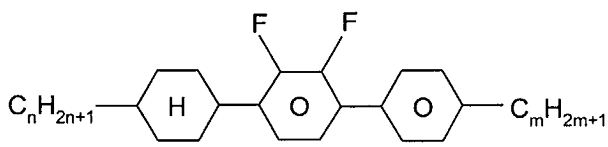

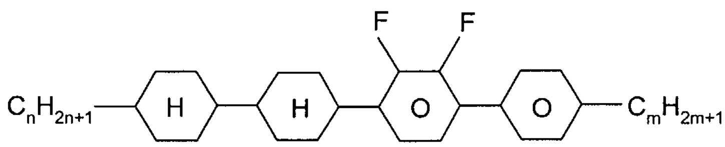

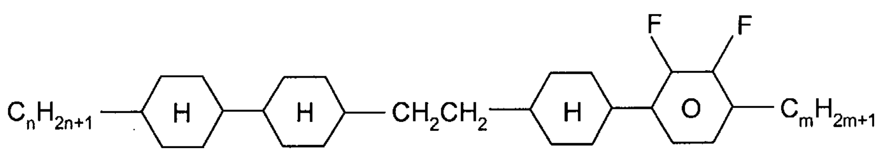

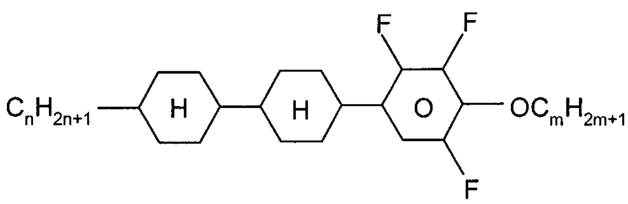

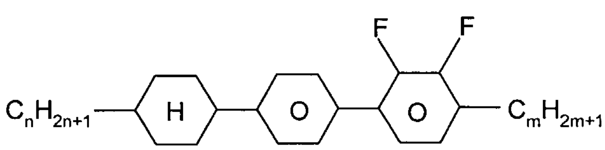

mit m, n = 1 bis 8.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formeln (I) und (II) eignen sich wegen ihres negativen $\Delta\varepsilon$ für eine Verwendung in VA-TFT-Displays. Gegenstand der Erfindung ist daher auch eine elektrooptische Flüssigkristallanzeige, enthaltend ein erfindungsgemäßes flüssigkristallines Medium.

Die Erfindung wird durch die nachstehenden Beispiele näher erläutert.

## Beispiele

Die Ausgangssubstanzen können nach allgemein zugänglichen Literaturvorschriften oder käuflich erhalten werden. Die beschriebenen Reaktionen sind literaturbekannt.

### Beispiel 1

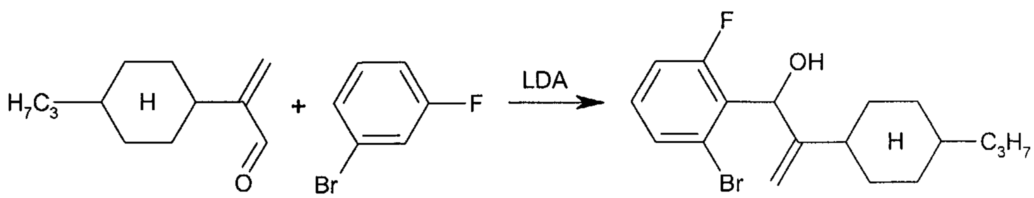


1 2 3

Bei -75°C werden 27,0 ml einer mit 100 ml THF verdünnten Lösung von 2N LDA in Cyclohexan/Ethylbenzol/ THF (52,4 mmol) mit einer Lösung von 7,0 ml (62,0 mmol) des Bromfluorbenzols **2** in 10 ml THF versetzt. Nach 2 Stunden bei der tiefen Temperatur werden 8,2 g (45,6 mmol) des Aldehyds 1 in 10 ml THF hinzugefügt. Nach 30 Minuten wird die Kühlung entfernt, und der Ansatz bei 20°C mit 100 ml 1N HCl versetzt. Nach Extraktion der wässrigen Phase, Trocknen der organischen Phase, Einengen und Chromatographie erhält man 13,5 g (83 % der Theorie) des Allylalkohols **3**.

### Beispiel 2

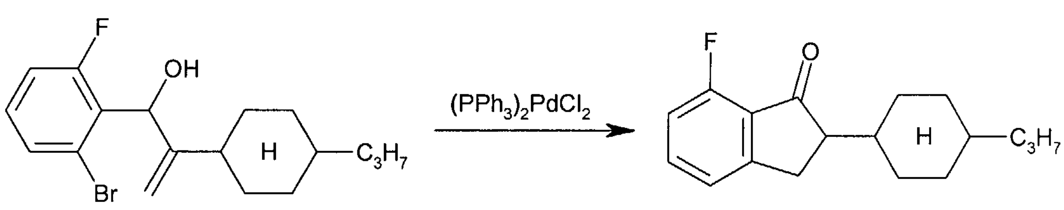


3 4

39,5 g (11,2 mmol) des Allylalkohols **3**, 5,5 g Bis(tri-o-tolylphosphin)palladiumdichlorid und 50 ml Triethylamin werden in 390 ml Acetonitril gelöst und bis zur vollständigen Umsetzung des Allylalkohols auf 90°C erwärmt. Der erkaltete Ansatz wird auf Wasser gegeben. Nach Extraktion, Trocknen, Einengen und Chromatographie erhält man 23,2 g (76 % der Theorie) des Indanons **4**.

## Beispiel 3

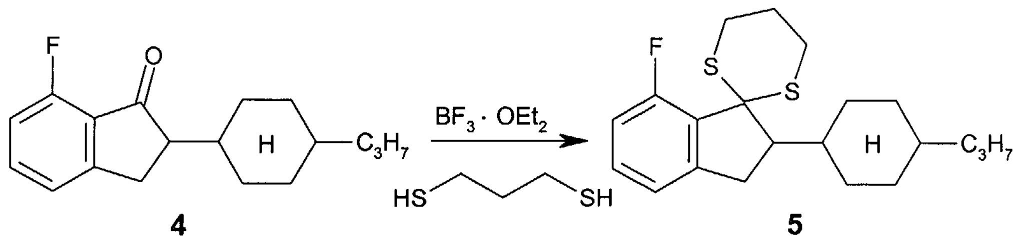


4 5

4,8 g (17,5 mmol) des Indanons **4** und 1,8 ml (17,6 mmol) Propandithiol werden in 30 ml Dichlormethan gelöst und bei 6 bis 7°C mit 4,0 ml Bortrifluorid-Diethylether-Komplex versetzt und anschließend über Nacht bei Raumtemperatur gerührt. Der Ansatz wird auf 10 ml gesättigte Natriumhydrogencarbonat-Lösung gegeben und bis zur Beendigung der Gasentwicklung gerührt. Nach Extraktion der wässrigen Phase, Trocknen der organischen Phase, Einengen und Filtration über Kieselgel wird der erhaltene Rückstand ohne weitere Reinigung in der nächsten Stufe eingesetzt.

## Beispiel 4

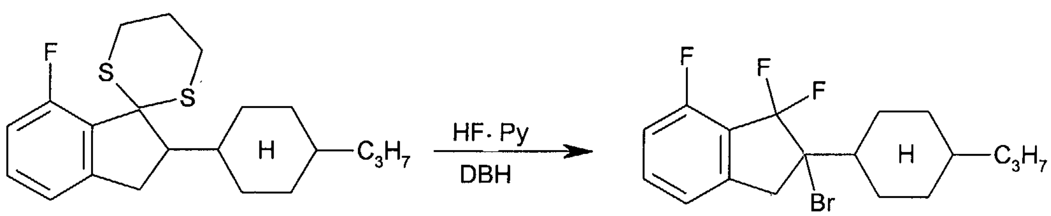


5 6

5,0 g des rohen Thioketals **5** in 25 ml Dichlormethan werden langsam bei -75°C in ein Gemisch aus 17,0 g (59,5 mmol) DBH, 60 ml einer 65 Gew.%-igen Lösung von HF in Pyridin und 35 ml Dichlormethan gegeben. Anschließend wird der Ansatz über Nacht bei Raumtemperatur gerührt. Das Reaktionsgemisch wird in eisgekühlte Hydrogensulfit-Lösung gegeben und mit gesättigter Natriumhydrogencarbonat-Lösung und Natronlauge entsäuert. Nach Extraktion, Trocknen, Einengen, erneutem Waschen mit Wasser, Chromatographie und Kristallisation aus Hexan erhält man 2,3 g des Trifluorbromindans **6**.

## Beispiel 5

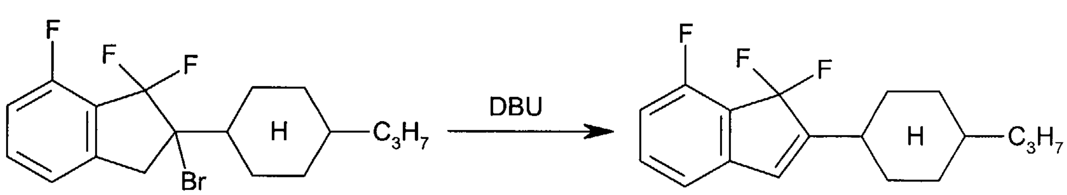


6 7

2,8 g (7,5 mmol) des Bromindans **6** werden in 25 ml Dichlormethan gelöst, mit 1,2 ml (8,0 mmol) DBU versetzt und bei Raumtemperatur gerührt, bis das Edukt vollständig umgesetzt ist. Der Ansatz wird mit Wasser und gesättigter Natriumchlorid-Lösung gewaschen, eingeengt und chromatographiert. Es werden 1,8 g (84 % der Theorie) des Indens **7** isoliert.

Spektroskopische Daten: ME (EI): M = 294
1H-NMR: 7,20 - 7,32 ppm, m (1H, Ar-H), 6,75 - 7,90 ppm; (2H, Ar-H); 6,32 ppm, s, (1H, 3-*H*).

Δε = -6,1
Δn = 0,107

## Beispiel 6

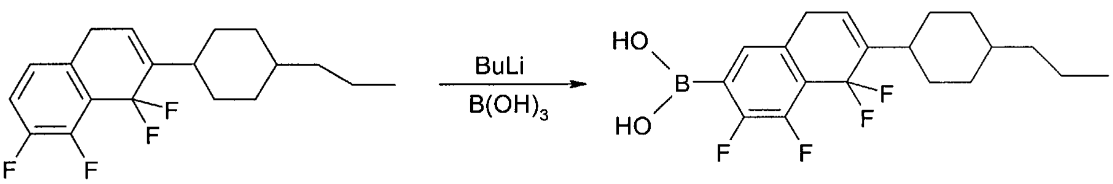


Unter Stickstoffatmosphäre werden 2,0 g (7,2 mmol) Inden in 50 ml THF gelöst und auf -70°C abgekühlt. Bei dieser Temperatur werden 4,5 ml (7,2 mmol) einer 15 %-igen Butyllithium-Lösung in n-Hexan in den Ansatz gegeben. Nach 1 Stunde werden 0,9 ml (8,0 mmol) Trimethylborat gelöst in 10 ml THF vorsichtig der Lösung zugesetzt. Der Ansatz wird auf 10°C erwärmt und hydrolysiert. Die Reaktionsmischung wird mit 2N HCl-Lösung angesäuert. Die wässrige Phase wird mit Methyl-tert.-Butylether extrahiert, die organische Phase getrocknet und eingeengt. Der erhaltene Rückstand wird ohne weitere Reinigung weiterverarbeitet.

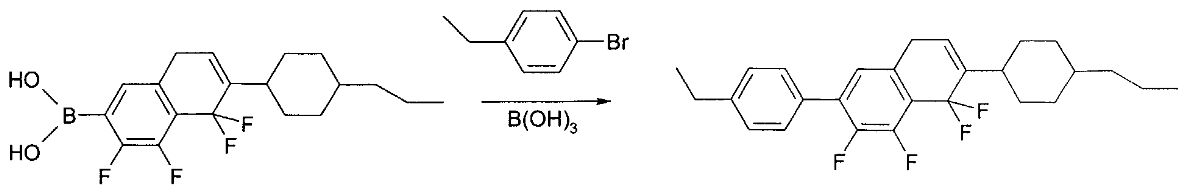


Unter Stickstoffatmosphäre werden 1,3 g (5,0 mmol) Natriummetaborat-Octahydrat in 2,0 ml Wasser gelöst und nacheinander mit 83 mg Bis(triphenylphosphin)-palladium(II)chlorid, 50 µl Hydraziniumhydroxid und 1,11 g (6,0 mmol) p-Brom-ethyl-benzol versetzt. Nach 5 Minuten bei Raumtemperatur wird der Ansatz mit 2,2 g (6,0 mmol) der Boronsäure gelöst in 3,75 ml THF versetzt und über Nacht zum Sieden erhitzt. Die wässrige Phase wird mit Methyl-tert.-Butylether extrahiert, die organische Phase wird mit Wasser gewaschen, getrocknet und eingeengt. Die Reinigung erfolgt mittels mehrfacher Chromatographie an Kieselgel (Heptan).

### Patentansprüche

1. Indene und 1,7-Dihydroindacene der allgemeinen Formel (I) bzw. (II) mit negativer dielektrischer Anisotropie (Δε < 0)

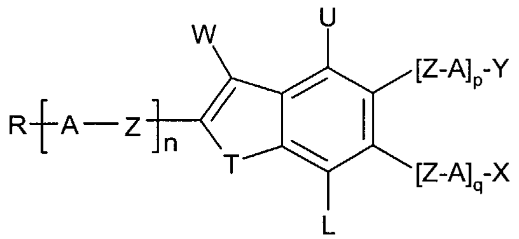

(I)

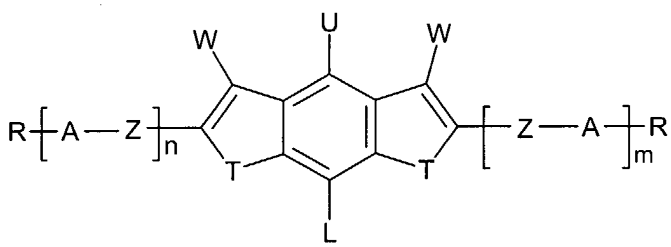

(II)

worin bedeuten:

T jeweils unabhängig voneinander
$>CF_2$ , $>CHF$ , $>CH_2$ , $>C=O$ ,

A jeweils unabhängig voneinander 1,4-Phenylen, worin =CH- ein- oder zweimal durch =N- ersetzt sein kann, und das ein- bis viermal unabhängig voneinander mit Halogen (-F, -Cl, -Br, -I), -CN, $-CH_3$, $-CH_2F$, $-CHF_2$, $-CF_3$, $-OCH_3$, $-OCH_2F$, $-OCHF_2$ oder $-OCF_3$ substituiert sein kann, 1,4-Cyclohexylen, 1,4-Cyclohexenylen oder 1,4-Cyclohexadienylen, worin $-CH_2-$ ein- oder zweimal unabhängig voneinander durch -O- oder -S- ersetzt sein kann, und die ein oder mehrfach durch Halogen substituiert sein können,

R jeweils unabhängig voneinander Wasserstoff, einen unsubstituierten, einen einfach durch $-CF_3$ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere $CH_2$-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN, -NCS, $-SF_5$, $-CF_3$, $-OCF_3$, $-OCHF_2$ oder $-OCH_2F$,

L Wasserstoff oder Halogen,

X Wasserstoff, einen unsubstituierten, einen einfach durch $-CF_3$ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere $CH_2$-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN oder -NCS,

Y, U, W unabhängig voneinander Wasserstoff, einen unsubstituierten, einen einfach durch $-CF_3$ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere $CH_2$-Gruppen jeweils unabhängig voneinander durch -O-, -S-, -CO-, -COO-, -OCO- oder -OCO-O- so ersetzt sein können, dass Heteroatome nicht direkt benachbart sind,

Z jeweils unabhängig voneinander eine Einfachbindung, $-CF_2O-$, $-OCF_2-$, $-CH_2CH_2-$, $-CF_2CF_2-$, -C(O)O-, -OC(O)-, $-CH_2O-$, $-OCH_2-$, -CF=CH-, -CH=CF-, -CF=CF-, -CH=CH-oder $-C\equiv C-$,

n, m unabhängig voneinander 0, 1, 2 oder 3,

p, q unabhängig voneinander 0, 1, 2 oder 3,

mit der Maßgabe, dass in Formel (I) L = -F ist, wenn T = $>CH_2$ ist und p und q jeweils 0 bedeuten.

2. Indene und 1,7-Dihydroindacene nach Anspruch 1, **dadurch gekennzeichnet, dass** in der allgemeinen Formel (I) bzw. (II)

A jeweils unabhängig voneinander

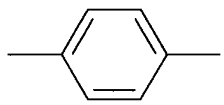 , 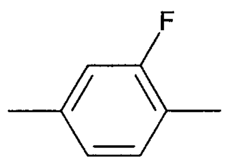 , 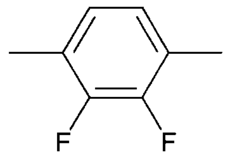 ,

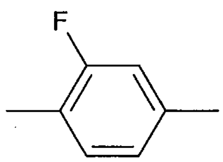 , 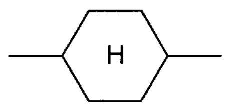 , 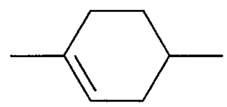 ,

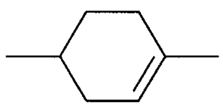 , 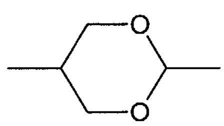 oder 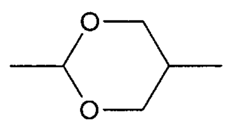

sind.

3. Indene und 1,7-Dihydroindacene nach Anspruch 1 oder 2, **dadurch gekennzeichnet, dass** in der allgemeinen Formel (I) bzw. (II)

R jeweils unabhängig voneinander ein Alkylrest, Alkoxyrest oder Alkenylrest mit 1 bis 7 bzw. 2 bis 7 C-Atomen sind.

4. Indene und 1,7-Dihydroindacene nach einem der Ansprüche 1 bis 3, **dadurch gekennzeichnet, dass** in der allgemeinen Formel (I) bzw. (II)

Z jeweils unabhängig voneinander eine Einfachbindung, $-CF_2O-$, $-OCF_2-$, $-CF_2CF_2-$, -CH=CH-, -CF=CH-, -CH=CF oder -CF=CF- sind.

5. Indene nach einem der Ansprüche 1 bis 4, **dadurch gekennzeichnet, dass** in der allgemeinen Formel (I)

X Wasserstoff, ein Alkylrest, Alkoxyrest oder Alkenylrest mit 1 bis 7 bzw. 2 bis 7 C-Atomen oder Halogen,

Y Wasserstoff ein Alkylrest, Alkoxyrest oder Alkenylrest mit 1 bis 7 bzw. 2 bis 7 C-Atomen und

U und W Wasserstoff

sind, und

n + p + q  1 oder 2 ist.

**6.** Dihydroindacene nach einem der Ansprüche 1 bis 4, **dadurch gekennzeichnet, dass** in der allgemeinen Formel (II)

U und W  Wasserstoff sind, und

m + n  1 oder 2 ist.

**7.** Flüssigkristallines Medium mit mindestens zwei flüssigkristallinen Verbindungen, enthaltend mindestens ein Inden und/oder 1,7-Dihydroindacen nach einem der Ansprüche 1 bis 6.

**8.** Elektrooptisches Anzeigeelement, enthaltend ein flüssigkristallines Medium nach Anspruch 7.

**9.** Verwendung von Indenen und 1,7-Dihydroindacenen, wie sie in einem der Ansprüche 1 bis 6 definiert sind, in flüssigkristallinen Medien für elektrooptische Anzeigeelemente.

Europäisches Patentamt

# EUROPÄISCHER RECHERCHENBERICHT

Nummer der Anmeldung
EP 03 00 4926

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | CHEMICAL ABSTRACTS, vol. 122, no. 16, 17. April 1995 (1995-04-17) Columbus, Ohio, US; abstract no. 201408, YOKOKOJI, OSAMU ET AL: "Fluorine-containing indane derivatives and liquid crystal compositions containing same" XP002248134 * Zusammenfassung * & JP 06 263663 A (ASAHI GLASS CO LTD, JAPAN) 20. September 1994 (1994-09-20) --- | 1,7-9 | C07C25/22<br>C07C49/697<br>C09K19/32 |
| A | PATENT ABSTRACTS OF JAPAN vol. 1998, no. 14, 31. Dezember 1998 (1998-12-31) & JP 10 236992 A (CHISSO CORP), 8. September 1998 (1998-09-08) * Zusammenfassung * --- | 1,7-9 | |
| A | DE 197 20 289 A (HOECHST AG) 19. November 1998 (1998-11-19) * das ganze Dokument * --- | 1,7-9 | RECHERCHIERTE SACHGEBIETE (Int.Cl.7)<br>C07C<br>C09K |
| P,A | WO 02 46330 A (MERCK PATENT GMBH) 13. Juni 2002 (2002-06-13) * das ganze Dokument * --- | 1,7-9 | |
| P,A | WO 02 079344 A (MERCK PATENT GMBH) 10. Oktober 2002 (2002-10-10) * das ganze Dokument * ----- | 1,7-9 | |

Der vorliegende Recherchenbericht wurde für alle Patentansprüche erstellt

| Recherchenort | Abschlußdatum der Recherche | Prüfer |
|---|---|---|
| DEN HAAG | 18. Juli 2003 | Beslier, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03 82 (P04C03)

## ANHANG ZUM EUROPÄISCHEN RECHERCHENBERICHT ÜBER DIE EUROPÄISCHE PATENTANMELDUNG NR. EP 03 00 4926

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-07-2003

| Im Recherchenbericht angeführtes Patentdokument | Datum der Veröffentlichung | Mitglied(er) der Patentfamilie | Datum der Veröffentlichung |
|---|---|---|---|
| JP 6263663 A | 20-09-1994 | KEINE | |
| JP 10236992 A | 08-09-1998 | KEINE | |
| DE 19720289 A | 19-11-1998 | DE 19720289 A1 | 19-11-1998 |
| WO 0246330 A | 13-06-2002 | AU 1392202 A | 18-06-2002 |
| | | DE 10155071 A1 | 08-08-2002 |
| | | WO 0246330 A1 | 13-06-2002 |
| WO 02079344 A | 10-10-2002 | DE 10115955 A1 | 10-10-2002 |
| | | WO 02079344 A1 | 10-10-2002 |

EPO FORM P0461

Für nähere Einzelheiten zu diesem Anhang : siehe Amtsblatt des Europäischen Patentamts, Nr.12/82